# EUROPEAN PATENT SPECIFICATION

(11) **EP 0 832 116 B1**
(45) Date of publication and mention of the grant of the patent: **17.09.2008**
(21) Application number: 96906991.3
(22) Date of filing: 12.03.1996
(51) Int. Cl.: C07K 14/35, G01N 33/53

(54) **NEW DNA MOLECULES**
DNA MOLEKÜLE
NOUVELLES MOLECULES D'ADN

(30) Priority: 30.05.1995 SE 9501976; 13.07.1995 SE 9502596; 19.09.1995 SE 9503246
(43) Date of publication of application: 01.04.1998
(73) Proprietor: AstraZeneca AB, 151 85 Södertälje (SE)
(72) Inventor: BALGANESH, Meenakshi, Bangalore 560094 (IN); SHARMA, Umender, Bangalore 560003 (IN)
(86) International application number: PCT/SE1996/000319
(87) International publication number: WO 1996/038478

(56) References cited:
- WO-A-95/17511
- ABSTRACTS OF THE GENERAL MEETING OF THE AMERICAN SOCIETY FOR MICROBIOLOGY, Vol. 94, 1994, D.M. WELTY et al., "Identification of a Putative rpoS Homologue from M. Marinum M. Tuberculosis, M. Ulcerans and M. Haemophilum", page 177.
- JOURNAL OF CELLULAR BIOCHEMISTRY SUPPLEMENT, Vol. 19B, 1995, T.S. BALGANESH et al., "B3201 Sigma Factors of M. Tuberculosis RNA Polymerase", page 73.
- MOLECULAR MICROBIOLOGY, Vol. 15, No. 2, 1995, MIMA PREDICH et al., "Characterization of RNA Polymerase and Two Sigma-Factor Genes from Mycobacterium Smegmatis", pages 355-366.

### SEQUENCE LISTING

(1) GENERAL INFORMATION:
   (i) APPLICANT:
      (A) NAME: Astra AB
      (B) STREET: Västra Mälarehamnen 9
      (C) CITY: Södertälje
      (E) COUNTRY: Sweden
      (F) POSTAL CODE (ZIP): S-151 85
      (G) TELEPHONE: +46-8-553 260 00
      (H) TELEFAX: +46-8-553 288 20
      (I) TELEX: 19237 astra s
   (ii) TITLE OF INVENTION: New DNA Molecules
   (iii) NUMBER OF SEQUENCES: 10
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 1724 base pairs
      (B) TYPE: nucleic acid
      (C) STRANDEDNESS: both
      (D) TOPOLOGY: linear
   (vi) ORIGINAL SOURCE:
      (A) ORGANISM: Mycobacterium tuberculosis
      (B) STRAIN: Erdman strain
   (vii) IMMEDIATE SOURCE:
      (B) CLONE: pARC 8175
   (ix) FEATURE:
      (A) NAME/KEY: CDS
      (B) LOCATION:70..1653
   (xi) SEQUENCE DESCRIPTION: SEQ ID NO: 1:
   AACTAGCAGA CACTTTCGGT TACGCACGCC CAGACCCAAC CGGAAGTGAG TAACGACCGA      60
   AGGGTGTAT GTG GCA GCG ACC AAA GCA AGC ACG GCG ACC GAT GAG CCG          108
             Val Ala Ala Thr Lys Ala Ser Thr Ala Thr Asp Glu Pro
               1               5                  10
   GTA AAA CGC ACC GCC ACC AAG TCG CCC GCG GCT TCC GCG TCC GGG GCC        156
   Val Lys Arg Thr Ala Thr Lys Ser Pro Ala Ala Ser Ala Ser Gly Ala
        15                  20                  25
   AAG ACC GGC GCC AAG CGA ACA GCG GCG AAG TCC GCT AGT GGC TCC CCA        204
   Lys Thr Gly Ala Lys Arg Thr Ala Ala Lys Ser Ala Ser Gly Ser Pro
    30                  35                  40                  45
   CCC GCG AAG CGG GCT ACC AAG CCC GCG GCC CGG TCC GTC AAG CCC GCC        252
   Pro Ala Lys Arg Ala Thr Lys Pro Ala Ala Arg Ser Val Lys Pro Ala
                    50                  55                  60
   TCG GCA CCC CAG GAC ACT ACG ACC AGC ACC ATC CCG AAA AGG AAG ACC      300
   Ser Ala Pro Gln Asp Thr Thr Thr Ser Thr Ile Pro Lys Arg Lys Thr
                65                  70                  75
   CGC GCC GCG GCC AAA TCC GCC GCC GCG AAG GCA CCG TCG GCC CGC GGC      348
   Arg Ala Ala Ala Lys Ser Ala Ala Ala Lys Ala Pro Ser Ala Arg Gly
            80                  85                  90
   CAC GCG ACC AAG CCA CGG GCG CCC AAG GAT GCC CAG CAC GAA GCC GCA      396
   His Ala Thr Lys Pro Arg Ala Pro Lys Asp Ala Gln His Glu Ala Ala
        95                 100                 105
   ACG GAT CCC GAG GAC GCC CTG GAC TCC GTC GAG GAG CTC GAC GCT GAA      444
   Thr Asp Pro Glu Asp Ala Leu Asp Ser Val Glu Glu Leu Asp Ala Glu
   110                 115                 120                 125
   CCA GAC CTC GAC GTC GAG CCC GGC GAG GAC CTC GAC CTT GAC GCC GCC      492
   Pro Asp Leu Asp Val Glu Pro Gly Glu Asp Leu Asp Leu Asp Ala Ala
                   130                 135                 140
   GAC CTC AAC CTC GAT GAC CTC GAG GAC GAC GTG GCG CCG GAC GCC GAC      540
   Asp Leu Asn Leu Asp Asp Leu Glu Asp Asp Val Ala Pro Asp Ala Asp
               145                 150                 155
   GAC GAC CTC GAC TCG GGC GAC GAC GAA GAC CAC GAA GAC CTC GAA GCT      588
   Asp Asp Leu Asp Ser Gly Asp Asp Glu Asp His Glu Asp Leu Glu Ala
           160                 165                 170
   GAG GCG GCC GTC GCG CCC GGC CAG ACC GCC GAT GAC GAC GAG GAG ATC      636
   Glu Ala Ala Val Ala Pro Gly Gln Thr Ala Asp Asp Asp Glu Glu Ile
       175                 180                 185
   GCT GAA CCC ACC GAA AAG GAC AAG GCC TCC GGT GAT TTC GTC TGG GAT      684
   Ala Glu Pro Thr Glu Lys Asp Lys Ala Ser Gly Asp Phe Val Trp Asp
   190                 195                 200                 205
   GAA GAC GAG TCG GAG GCC CTG CGT CAA GCA CGC AAG GAC GCC GAA CTC      732
   Glu Asp Glu Ser Glu Ala Leu Arg Gln Ala Arg Lys Asp Ala Glu Leu
                   210                 215                 220
   ACC GCA TCC GCC GAC TCG GTT CGC GCC TAC CTC AAA CAG ATC GGC AAG      780
   Thr Ala Ser Ala Asp Ser Val Arg Ala Tyr Leu Lys Gln Ile Gly Lys
               225                 230                 235
   GTA GCG CTG CTC AAC GCC GAG GAA GAG GTC GAG CTA GCC AAG CGG ATC      828
   Val Ala Leu Leu Asn Ala Glu Glu Glu Val Glu Leu Ala Lys Arg Ile
           240                 245                 250
   GAG GCT GGC CTG TAC GCC ACG CAG CTG ATG ACC GAG CTT AGC GAG CGC      876
   Glu Ala Gly Leu Tyr Ala Thr Gln Leu Met Thr Glu Leu Ser Glu Arg
       255                 260                 265
   GGC GAA AAG CTG CCT GCC GCC CAG CGC CGC GAC ATG ATG TGG ATC TGC      924
   Gly Glu Lys Leu Pro Ala Ala Gln Arg Arg Asp Met Met Trp Ile Cys
   270                 275                 280                 285
   CGC GAC GGC GAT CGC GCG AAA AAC CAT CTG CTG GAA GCC AAC CTG CGC      972
   Arg Asp Gly Asp Arg Ala Lys Asn His Leu Leu Glu Ala Asn Leu Arg
                   290                 295                 300
   CTG GTG GTT TCG CTA GCC AAG CGC TAC ACC GGC CGG GGC ATG GCG TTT     1020
   Leu Val Val Ser Leu Ala Lys Arg Tyr Thr Gly Arg Gly Met Ala Phe
               305                 310                 315
   CTC GAC CTG ATC CAG GAA GGC AAC CTG GGG CTG ATC CGC GCG GTG GAG     1068
   Leu Asp Leu Ile Gln Glu Gly Asn Leu Gly Leu Ile Arg Ala Val Glu
           320                 325                 330
   AAG TTC GAC TAC ACC AAG GGG TAC AAG TTC TCC ACC TAC GCT ACG TGG     1116
   Lys Phe Asp Tyr Thr Lys Gly Tyr Lys Phe Ser Thr Tyr Ala Thr Trp
       335                 340                 345
   TGG ATT CGC CAG GCC ATC ACC CGC GCC ATG GCC GAC CAG GCC CGC ACC     1164
   Trp Ile Arg Gln Ala Ile Thr Arg Ala Met Ala Asp Gln Ala Arg Thr
   350                 355                 360                 365
   ATC CGC ATC CCG GTG CAC ATG GTC GAG GTG ATC AAC AAG CTG GGC CGC     1212
   Ile Arg Ile Pro Val His Met Val Glu Val Ile Asn Lys Leu Gly Arg
                   370                 375                 380
   ATT CAA CGC GAG CTG CTG CAG GAC CTG GGC CGC GAG CCC ACG CCC GAG     1260
   Ile Gln Arg Glu Leu Leu Gln Asp Leu Gly Arg Glu Pro Thr Pro Glu
               385                 390                 395
   GAG CTG GCC AAA GAG ATG GAC ATC ACC CCG GAG AAG GTG CTG GAA ATC     1308
   Glu Leu Ala Lys Glu Met Asp Ile Thr Pro Glu Lys Val Leu Glu Ile
           400                 405                 410
   CAG CAA TAC GCC CGC GAG CCG ATC TCG TTG GAC CAG ACC ATC GGC GAC     1356
   Gln Gln Tyr Ala Arg Glu Pro Ile Ser Leu Asp Gln Thr Ile Gly Asp
       415                 420                 425
   GAG GGC GAC AGC CAG CTT GGC GAT TTC ATC GAA GAC AGC GAG GCG GTG     1404
   Glu Gly Asp Ser Gln Leu Gly Asp Phe Ile Glu Asp Ser Glu Ala Val
   430                 435                 440                 445
   GTG GCC GTC GAC GCG GTG TCC TTC ACT TTG CTG CAG GAT CAA CTG CAG     1452
   Val Ala Val Asp Ala Val Ser Phe Thr Leu Leu Gln Asp Gln Leu Gln
                   450                 455                 460
   TCG GTG CTG GAC ACG CTC TCC GAG CGT GAG GCG GGC GTG GTG CGG CTA     1500
   Ser Val Leu Asp Thr Leu Ser Glu Arg Glu Ala Gly Val Val Arg Leu
               465                 470                 475
   CGC TTC GGC CTT ACC GAC GGC CAG CCG CGC ACC CTT GAC GAG ATC GGC     1548
   Arg Phe Gly Leu Thr Asp Gly Gln Pro Arg Thr Leu Asp Glu Ile Gly
           480                 485                 490
   CAG GTC TAC GGC GTG ACC CGG GAA CGC ATC CGC CAG ATC GAA TCC AAG     1596
   Gln Val Tyr Gly Val Thr Arg Glu Arg Ile Arg Gln Ile Glu Ser Lys
       495                 500                 505
   ACT ATG TCG AAG TTG CGC CAT CCG AGC CGC TCA CAG GTC CTG CGC GAC     1644
   Thr Met Ser Lys Leu Arg His Pro Ser Arg Ser Gln Val Leu Arg Asp
   510                 515                 520                 525
   TAC CTG GAC TGAGAGCGCC CGCCGAGGCG ACCAACGTAG CACGTGAGCC             1693
   Tyr Leu Asp
   CCCAGCAGCT AGCCGCACCA TGGTCTCGTC C                                  1724
(2) INFORMATION FOR SEQ ID NO: 2:
   (i) SEQUENCE CHARACTERISTICS:
      (A) LENGTH: 528 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Val Ala Ala Thr Lys Ala Ser Thr Ala Thr Asp Glu Pro Val Lys Arg
    1               5                   10                  15
   Thr Ala Thr Lys Ser Pro Ala Ala Ser Ala Ser Gly Ala Lys Thr Gly
               20                  25                  30
   Ala Lys Arg Thr Ala Ala Lys Ser Ala Ser Gly Ser Pro Pro Ala Lys
           35                  40                  45
   Arg Ala Thr Lys Pro Ala Ala Arg Ser Val Lys Pro Ala Ser Ala Pro
       50                  55                  60
   Gln Asp Thr Thr Thr Ser Thr Ile Pro Lys Arg Lys Thr Arg Ala Ala
   65                  70                  75                  80
   Ala Lys Ser Ala Ala Ala Lys Ala Pro Ser Ala Arg Gly His Ala Thr
                   85                  90                  95
   Lys Pro Arg Ala Pro Lys Asp Ala Gln His Glu Ala Ala Thr Asp Pro
               100                 105                 110
   Glu Asp Ala Leu Asp Ser Val Glu Glu Leu Asp Ala Glu Pro Asp Leu
           115                 120                 125
   Asp Val Glu Pro Gly Glu Asp Leu Asp Leu Asp Ala Ala Asp Leu Asn
       130                 135                 140
   Leu Asp Asp Leu Glu Asp Asp Val Ala Pro Asp Ala Asp Asp Asp Leu
   145                 150                 155                 160
   Asp Ser Gly Asp Asp Glu Asp His Glu Asp Leu Glu Ala Glu Ala Ala
                   165                 170                 175
   Val Ala Pro Gly Gln Thr Ala Asp Asp Asp Glu Glu Ile Ala Glu Pro
               180                 185                 190
   Thr Glu Lys Asp Lys Ala Ser Gly Asp Phe Val Trp Asp Glu Asp Glu
           195                 200                 205
   Ser Glu Ala Leu Arg Gln Ala Arg Lys Asp Ala Glu Leu Thr Ala Ser
       210                 215                 220
   Ala Asp Ser Val Arg Ala Tyr Leu Lys Gln Ile Gly Lys Val Ala Leu
   225                 230                 235                 240
   Leu Asn Ala Glu Glu Glu Val Glu Leu Ala Lys Arg Ile Glu Ala Gly
                   245                 250                 255
   Leu Tyr Ala Thr Gln Leu Met Thr Glu Leu Ser Glu Arg Gly Glu Lys
               260                 265                 270
   Leu Pro Ala Ala Gln Arg Arg Asp Met Met Trp Ile Cys Arg Asp Gly
           275                 280                 285
   Asp Arg Ala Lys Asn His Leu Leu Glu Ala Asn Leu Arg Leu Val Val
       290                 295                 300
   Ser Leu Ala Lys Arg Tyr Thr Gly Arg Gly Met Ala Phe Leu Asp Leu
   305                 310                 315                 320
   Ile Gln Glu Gly Asn Leu Gly Leu Ile Arg Ala Val Glu Lys Phe Asp
                   325                 330                 335
   Tyr Thr Lys Gly Tyr Lys Phe Ser Thr Tyr Ala Thr Trp Trp Ile Arg
               340                 345                 350
   Gln Ala Ile Thr Arg Ala Met Ala Asp Gln Ala Arg Thr Ile Arg Ile
           355                 360                 365
   Pro Val His Met Val Glu Val Ile Asn Lys Leu Gly Arg Ile Gln Arg
       370                 375                 380
   Glu Leu Leu Gln Asp Leu Gly Arg Glu Pro Thr Pro Glu Glu Leu Ala
   385                 390                 395                 400
   Lys Glu Met Asp Ile Thr Pro Glu Lys Val Leu Glu Ile Gln Gln Tyr
                   405                 410                 415
   Ala Arg Glu Pro Ile Ser Leu Asp Gln Thr Ile Gly Asp Glu Gly Asp
               420                 425                 430
   Ser Gln Leu Gly Asp Phe Ile Glu Asp Ser Glu Ala Val Val Ala Val
           435                 440                 445
   Asp Ala Val Ser Phe Thr Leu Leu Gln Asp Gln Leu Gln Ser Val Leu
       450                 455                 460
   Asp Thr Leu Ser Glu Arg Glu Ala Gly Val Val Arg Leu Arg Phe Gly
   465                 470                 475                 480
   Leu Thr Asp Gly Gln Pro Arg Thr Leu Asp Glu Ile Gly Gln Val Tyr
                   485                 490                 495
   Gly Val Thr Arg Glu Arg Ile Arg Gln Ile Glu Ser Lys Thr Met Ser
               500                 505                 510
   Lys Leu Arg His Pro Ser Arg Ser Gln Val Leu Arg Asp Tyr Leu Asp
           515                 520                 525
(2) INFORMATION FOR SEQ ID NO: 3:
   (i) SEQUENCE CHARACTERISTICS:
      (A) LENGTH: 1508 base pairs
      (B) TYPE: nucleic acid
      (C) STRANDEDNESS: both
      (D) TOPOLOGY: linear
   (vi) ORIGINAL SOURCE:
      (A) ORGANISM: Mycobacterium tuberculosis
      (C) INDIVIDUAL ISOLATE: atcc27294
   (vii) IMMEDIATE SOURCE:
      (B) CLONE: pARC 8176
   (ix) FEATURE:
      (A) NAME/KEY: CDS
      (B) LOCATION:325..1293
   (xi) SEQUENCE DESCRIPTION: SEQ ID NO: 3:
   ACCAGCCCGA CGACCGACGA ACCCCGCCGC TTCGACGTGC CCAGCCGGCG CATCCCGCTG     60
   TTCCCGACCG CGAACGGCCC GCACTCGAGC CGACGGCGAC AGCCGGCAAG AAGCGGTCAG    120
   CCCGCGGGGA TTCGCCGACC ACGGTTAGCC GTCTGTTGGC CGGCGTTCCG GGTTGTCGCC    180
   ACTGGCCACA CTTCTCAGGA CTTTCTCAGG TCTTCGGCAG ATTCCTGCAC GTCACAGGGC    240
   GTCAGATCAC TGCTGGGTGG GAACTCAAAG TCCGGCTTTG TCGTTAAACC CTGACAGTGC    300
   AAGCCGATCG GGGAACGGCT CGCT ATG GCC GAT GCA CCC ACA AGG GCC ACC       351
                              Met Ala Asp Ala Pro Thr Arg Ala Thr
                                  530                 535
   ACA AGC CGG GTT GAC ACA GAT CTG GAT GCT CAA AGC CCC GCG GCG GAC      399
   Thr Ser Arg Val Asp Thr Asp Leu Asp Ala Gln Ser Pro Ala Ala Asp
           540                 545                 550
   CTC GTG CGC GTC TAT CTG AAC GGC ATC GGC AAG ACG GCG TTG CTC AAC      447
   Leu Val Arg Val Tyr Leu Asn Gly Ile Gly Lys Thr Ala Leu Leu Asn
       555                 560                 565
   GCG GCG GAT GAA GTC GAA CTG GCC AAG CGC ATA GAA GCC GGG TTG TAT      495
   Ala Ala Asp Glu Val Glu Leu Ala Lys Arg Ile Glu Ala Gly Leu Tyr
   570                 575                 580                 585
   GCC GAG CAT CTG CTG GAA ACC CGG AAG CGC CTC GGC GAG AAC CGA AAA      543
   Ala Glu His Leu Leu Glu Thr Arg Lys Arg Leu Gly Glu Asn Arg Lys
                   590                 595                 600
   CGC GAC CTG GCG GCC GTG GTG CGT GAT GGC GAG GCC GCC CGC CGC CAC      591
   Arg Asp Leu Ala Ala Val Val Arg Asp Gly Glu Ala Ala Arg Arg His
               605                 610                 615
   CTG CTG GAA GCA AAC CTG CGG CTG GTG GTA TCG CTG GCC AAG CGC TAC      639
   Leu Leu Glu Ala Asn Leu Arg Leu Val Val Ser Leu Ala Lys Arg Tyr
           620                 625                 630
   ACG GGT CGG GGC ATG CCG TTG CTG GAC CTC ATC CAG GAG GGC AAC CTG      687
   Thr Gly Arg Gly Met Pro Leu Leu Asp Leu Ile Gln Glu Gly Asn Leu
       635                 640                 645
   GGT CTG ATC CGA GCG ATG GAG AAG TTC GAC TAC ACA AAG GGA TTC AAG      735
   Gly Leu Ile Arg Ala Met Glu Lys Phe Asp Tyr Thr Lys Gly Phe Lys
   650                 655                 660                 665
   TTC TCA ACG TAT GCC ACG TGG TGG ATC CGC CAG GCC ATC ACC CGC GGA      783
   Phe Ser Thr Tyr Ala Thr Trp Trp Ile Arg Gln Ala Ile Thr Arg Gly
                   670                 675                 680
   ATG GCC GAC CAG AGC CGC ACC ATC CGC CTG CCC GTA CAC CTG GTT GAG      831
   Met Ala Asp Gln Ser Arg Thr Ile Arg Leu Pro Val His Leu Val Glu
               685                 690                 695
   CAG GTC AAC AAG CTG GCG CGG ATC AAG CGG GAG ATG CAC CAG CAT CTG      879
   Gln Val Asn Lys Leu Ala Arg Ile Lys Arg Glu Met His Gln His Leu
           700                 705                 710
   GGT CGC GAA CGC ACC GAT GAG GAG CTC GCC GCC GAA TCC GGC ATT CCA      927
   Gly Arg Glu Arg Thr Asp Glu Glu Leu Ala Ala Glu Ser Gly Ile Pro
       715                 720                 725
   ATC GAC AAG ATC AAC GAC CTG CTG GAA CAC AGT CGC GAC CCG GTG AGT      975
   Ile Asp Lys Ile Asn Asp Leu Leu Glu His Ser Arg Asp Pro Val Ser
   730                 735                 740                 745
   CTG GAT ATG CCG GTC GGC TCC GAG GAG GAG GCC CCT TTG GGC GAT TTC     1023
   Leu Asp Met Pro Val Gly Ser Glu Glu Glu Ala Pro Leu Gly Asp Phe
                   750                 755                 760
   ATC GAG GAC GCC GAA GCC ATG TCC GCG GAG AAC GCG GTC ATC GCC GAA     1071
   Ile Glu Asp Ala Glu Ala Met Ser Ala Glu Asn Ala Val Ile Ala Glu
               765                 770                 775
   CTG TTA CAC ACC GAC ATC CGC AGC GTG CTG GCC ACT CTC GAC GAG CGT     1119
   Leu Leu His Thr Asp Ile Arg Ser Val Leu Ala Thr Leu Asp Glu Arg
           780                 785                 790
   GAC GAC CAG GTG ATC CGG CTG CGC TTC GGC CTG GAT GAC GGC CAA CCA     1167
   Asp Asp Gln Val Ile Arg Leu Arg Phe Gly Leu Asp Asp Gly Gln Pro
       795                 800                 805
   CGC ACC CTG GAT CAA ATC GGC AAA CTA TTC GGG CTG TCC CGT GAG CGG    1215
   Arg Thr Leu Asp Gln Ile Gly Lys Leu Phe Gly Leu Ser Arg Glu Arg
   810                 815                 820                 825
   GTT CGT CAG ATC GAG CGC GAC GTG ATG AGT AAG CTG CGG CAC GGT GAG    1263
   Val Arg Gln Ile Glu Arg Asp Val Met Ser Lys Leu Arg His Gly Glu
                   830                 835                 840
   CGG GCG GAT CGG CTG CGG TCG TAC GCC AGC TGAAGCTGGA CATCCTGAGC      1313
   Arg Ala Asp Arg Leu Arg Ser Tyr Ala Ser
               845                 850
   CAGGTAGCAG ACGGTATGCC CGCCGCGCCA GCATAGCCTG CGGTGGGGCG GCGGGCAACC  1373
   ATTTTCGCAG CTGGCCAAGT GTAGACTCAG CTGCAATGGA GGGTGCTGAA TGAACGAGTT  1433
   GGTTGATACC ACCGAGATGT ACCTGCGGAC CATCTACGAC CTCGAGGAAG AGGGCGTGAC  1493
   GCACTGCGTG CCGGA                                                   1508
(2) INFORMATION FOR SEQ ID NO: 4:
   (i) SEQUENCE CHARACTERISTICS:
      (A) LENGTH: 323 amino acids
      (B) TYPE: amino acid
      (D) TOPOLOGY: linear
   (ii) MOLECULE TYPE: protein
   (xi) SEQUENCE DESCRIPTION: SEQ ID NO: 4:
   Met Ala Asp Ala Pro Thr Arg Ala Thr Thr Ser Arg Val Asp Thr Asp
    1               5                  10                  15
   Leu Asp Ala Gln Ser Pro Ala Ala Asp Leu Val Arg Val Tyr Leu Asn
               20                  25                  30
   Gly Ile Gly Lys Thr Ala Leu Leu Asn Ala Ala Asp Glu Val Glu Leu
           35                  40                  45
   Ala Lys Arg Ile Glu Ala Gly Leu Tyr Ala Glu His Leu Leu Glu Thr
       50                  55                  60
   Arg Lys Arg Leu Gly Glu Asn Arg Lys Arg Asp Leu Ala Ala Val Val
   65                  70                  75                  80
   Arg Asp Gly Glu Ala Ala Arg Arg His Leu Leu Glu Ala Asn Leu Arg
                   85                  90                  95
   Leu Val Val Ser Leu Ala Lys Arg Tyr Thr Gly Arg Gly Met Pro Leu
               100                 105                 110
   Leu Asp Leu Ile Gln Glu Gly Asn Leu Gly Leu Ile Arg Ala Met Glu
           115                 120                 125
   Lys Phe Asp Tyr Thr Lys Gly Phe Lys Phe Ser Thr Tyr Ala Thr Trp
       130                 135                 140
   Trp Ile Arg Gln Ala Ile Thr Arg Gly Met Ala Asp Gln Ser Arg Thr
   145                 150                 155                 160
   Ile Arg Leu Pro Val His Leu Val Glu Gln Val Asn Lys Leu Ala Arg
                   165                 170                 175
   Ile Lys Arg Glu Met His Gln His Leu Gly Arg Glu Arg Thr Asp Glu
               180                 185                 190
   Glu Leu Ala Ala Glu Ser Gly Ile Pro Ile Asp Lys Ile Asn Asp Leu
           195                 200                 205
   Leu Glu His Ser Arg Asp Pro Val Ser Leu Asp Met Pro Val Gly Ser
       210                 215                 220
   Glu Glu Glu Ala Pro Leu Gly Asp Phe Ile Glu Asp Ala Glu Ala Met
   225                 230                 235                 240
   Ser Ala Glu Asn Ala Val Ile Ala Glu Leu Leu His Thr Asp Ile Arg
                   245                 250                 255
   Ser Val Leu Ala Thr Leu Asp Glu Arg Asp Asp Gln Val Ile Arg Leu
               260                 265                 270
   Arg Phe Gly Leu Asp Asp Gly Gln Pro Arg Thr Leu Asp Gln Ile Gly
           275                 280                 285
   Lys Leu Phe Gly Leu Ser Arg Glu Arg Val Arg Gln Ile Glu Arg Asp
       290                 295                 300
   Val Met Ser Lys Leu Arg His Gly Glu Arg Ala Asp Arg Leu Arg Ser
   305                 310                 315                 320
   Tyr Ala Ser
(2) INFORMATION FOR SEQ ID NO: 5:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO: 5:
      AAGTTCAGCA CSTACGCSAC STGGTGGATC 30
(2) INFORMATION FOR SEQ ID NO: 6:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO: 6:
      CTTSGCCTCG ATCTGSCGGA TSCGCTC 27
(2) INFORMATION FOR SEQ ID NO: 7:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO: 7:
      TTCCATGGGG TATGTGGCAG CGACC 25
(2) INFORMATION FOR SEQ ID NO: 8:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO: 8:
      GTACAGGCCA CCCTCCATCC GCTTGCC 27
(2) INFORMATION FOR SEQ ID NO: 9:
   (i) SEQUENCE CHARACTERISTICS:
      (A) LENGTH: 28 base pairs
      (B) TYPE: nucleic acid
      (C) STRANDEDNESS: single
      (D) TOPOLOGY: .linear
   (ii) MOLECULE TYPE: other nucleic acid
      (A) DESCRIPTION: /desc = "PCR primer"
   (xi) SEQUENCE DESCRIPTION: SEQ ID NO: 9:
      TTTCATGGCC GATGCACCCA CAAGGGCC 28
(2) INFORMATION FOR SEQ ID NO: 10:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO: 10:
      CTTGAATTCA CCTCGCCTAC GACCGCA 27

## Description

### TECHNICAL FIELD

The present invention provides novel nucleic acid molecules coding for sigma subunits of *Mycobacterium tuberculosis* RNA polymerase. It also relates to polypeptides, referred to as SigB, encoded by such nucleic acid molecules, as well as to vectors and host cells transformed with the said nucleic acid molecules. The invention further provides screening assays for compounds which inhibit the interaction between a sigma subunit and a core RNA polymerase.

### BACKGROUND ART

Transcription of genes to the corresponding RNA molecules is a complex process which is catalyzed by DNA dependent RNA polymerase, and involves many different protein factors. In eubacteria, the core RNA polymerase, is composed of α, β, and β' subunits in the ratio 2:1:1. To direct RNA polymerase to promoters of specific genes to be transcribed, bacteria produce a variety of proteins, known as sigma (σ) factors, which interact with RNA polymerase to form an active holoenzyme. The resulting complexes arc able to recognize and attach to selected nucleotide sequences in promoters.

Physical measurements have shown that the sigma subunit induces conformational transition upon binding to the core RNA polymerase. Binding of the sigma subunit to the core enzyme increases the binding constant of the core enzyme for DNA by several orders of magnitude (Chamberlin, M.J. (1974) Ann. Rev. Biochcm. 43, 721-).

Characterisation of sigma subunits, identified and sequenced from various organisms, allows them to be classified into two broad categories; Group I and Group II. The Group I sigma has also been referred to as the sigma⁷⁰ class, or the "house keeping" sigma group. Sigma subunits belonging to this group recognise similar promoter sequences in the cell. These properties are reflected in certain regions of the proteins which are highly conserved between species.

Bacterial sigma factors do not have any homology with eukaryotic transcription factors, and arc consequently a potential target for antibacterial compounds. Mutations in the sigma subunit, effecting its association and ability to confer DNA sequence specificity to the enzyme, are known to be lethal to the cell.

*Mycobacterium tuberculosis* is a major pulmonary pathogen which is characterized by its very slow growth rate. As a pathogen it gains access to alveolar macrophages where it multiplies within the phagosome, finally lysing the cells and being disseminated through the blood stream, not only to other areas of the lung, but also to extrapulmonary tissues. Thus the pathogen multiplies in at least two entirely different environments, which would involve the utilisation of different nutrients and a variety of possible host factors; a successful infection would thus involve the coordinated expression of new sets of genes. This regulation would resemble different physiological stages, as best exemplified by *Bacillus,* in which the expression of genes specific for different stages are transcribed by RNA polymerases associating with different sigma factors. This provides the possibility of targeting not only the house keeping sigma of *M. tuberculosis,* but also sigma subunits specific for the different stages of infection and dissemination.

WO-95/17511 discloses sigma A polypeptides and nucleic acids from *M. tuberculosis*

### BRIEF DESCRIPTION OF THE DRAWINGS

Fig. 1: Map of plasmid pARC 8175
Fig. 2: Map of plasmid pARC 8176

### PURPOSE OF THE INVENTION

Since the association to a specific sigma subunit is essential for the specificity of RNA polymerase, this process of association is a suitable target for drug design. In order to identify compounds capable of inhibiting the said association process, the identification of the primary structures of sigma subunits is desirable.

It is thus the purpose of the invention to provide information on sequences and structure of sigma subunits, which information will enable the screening, identification and design of compounds competing with the sigma subunit for binding to the core RNA polymerase, which compounds may be developed into effective therapeutic agent.

### DISCLOSURE OF THE INVENTION

Throughout this description and in particular in the following examples, the therms "standard protocols" and "standard procedures", when used in the context of molecular cloning techniques, are to be understood as protocols and procedures found in an ordinary laboratory manual such as: Sambrook, J., Fritsch, E.F. and Maniatis, T. (1989) Molecular Cloning: A laboratory manual, 2nd Ed., Cold Spring Harbor Laboratory Press, Cold Spring Harbor, NY.

We provide an isolated polypeptide which is a Group I sigma subunit of *Mycobacterium tuberculosis* RNA polymerase, or a functionally equivalent modified form thereof.

Polypeptides having amino acid sequences according to SEQ ID NO: 2 or 4 of the Sequence Listing have been obtained by recombinant DNA techniques and are hereinafter referred to as SigA and SigB polypeptides. However, it will be understood that the polypeptides according to the invention are not limited strictly to polypeptides with an amino acid sequence identical with SEQ ID NO: 4 in the Sequence Listing. Rather the invention additionally encompasses modified forms of these native polypeptides carrying modifications like substitutions, small deletions, insertions or inversions, which polypeptides nevertheless have substantially the biological activities of a *M. tuberculosis* sigma subunit. Such biological activities comprise the ability to associate with the core enzyme and / or confer the property of promoter sequence recognition and initiation of transcription. Included in the invention are consequently polypeptides, the amino acid sequence of which are at least 90% homologous, preferably at least 95% homologous, with the amino acid sequence shown as SEQ ID NO: 4 in the Sequence Listing.

In another aspect, the invention provides isolated and purified nucleic acid molecules which have a nucleotide sequence coding for a polypeptide of the invention e.g. the SigB polypeptide. In a preferred form of the invention, the said nucleic acid molecules are DNA molecules which have a nucleotide sequence identical with SEQ ID NO: 3 of the Sequence Listing. However, the nucleic acid molecules according to the invention are not to be limited strictly to the DNA molecules with the sequence shown as SEQ ID NO: 3. Rather the invention encompasses nucleic acid molecules carrying modifications like substitutions, small deletions, insertions or inversions, which nevertheless encode proteins having substantially the biochemical activity of the polypeptides according to the invention. Included in the invention are consequently DNA molecules, the nucleotide sequences of which are at least 90% homologous, preferably at least 95% homologous, with the nucleotide sequence shown as SEQ ID NO: 3 in the Sequence Listing.

Included in the invention are also DNA molecule which nucleotide sequences are degenerate, because of the genetic code, to the nucleotide sequences shown as SEQ ID NO:3. A sequential grouping of three nucleotides, a "codon", codes for one amino acid. Since there are 64 possible codons, hut only 20 natural amino acids, most amino acids are coded for by more than one codon. This natural "degeneracy", or "redundancy", of the genetic code is well known in the art. It will thus be appreciated that the DNA sequence shown in the Sequence Listing is only an example within a large but definite group of DNA sequences which will encode the polypeptide as described above.

Included in the invention are consequently isolated nucleic acid molecule selected from:
(a) DNA molecules comprising a nucleotide sequence as shown in SEQ ID NO: 3 encoding a Group I sigma subunit of *Mycobacterium tuberculosis* RNA polymerase; and
(b) nucleic acid molecules comprising a nucleic acid sequence which is degenerate, as a result of the genetic code, to a nucleotide sequence as defined in (a) or (h) and which codes for a polypeptide which is a Group I sigma subunit of *Mycobacterium tuberculosis* or a functionally equivalent modified form thereof.

The term "hybridizing to a nucleotide sequence" should be understood as hybridizing to a nucleotide sequence, or a specific part thereof, under stringent hybridization conditions which are known to a person skilled in the art.

A DNA molecules of the invention may be in the form of a vector, e.g. a replicable expression vector which carries and is capable of mediating the expression of a DNA molecule according to the invention. In the present context the term "replicable" means that the vector is able to replicate in a given type of host cell into which is has been introduced. Examples of vectors are viruses such as bacteriophages, cosmids, plasmids and other recombination vectors. Nucleic acid molecules are inserted into vector genomes by methods well known in the art. Vectors according to the invention can include the plasmid vector pARC 8176 (NCIMB 40739) which contains the coding sequence of the *sigB* gene.

Included in the invention is also a host cell harbouring a vector according to the invention. Such a host cell can be a prokaryotic cell, a unicellular eukaryotic cell or a cell derived from a multicellular organism. The host cell can thus e.g. be a bacterial cell such as an *E. coli* cell; a cell from a yeast such as *Saccharomyces cervisiae* or . *Pichia pastoris,* or a mammalian cell. The methods employed to effect introduction of the vector into the host cell are standard methods well known to a person familiar with recombinant DNA methods.

A further aspect of the invention is a process for production of a polypeptide of the invention, comprising culturing host cells transformed with an expression vector according of the invention under conditions whereby said polypeptide is produced, and recovering said polypeptide.

The medium used to grow the cells may be any conventional medium suitable for the purpose. A suitable vector may be any of the vectors described above, and an appropriate host cell may be any of the cell types listed above. The method employed to construct the vector and effect introduction thereof into the host cell may be any methods known for such purposes within the field of recombinant DNA. The recombinant polypeptide expressed by the cells may be secreted, i.e. exported through the cell membrane, dependent on the type of cell and the composition of the vector.

If the polypeptide is produced intracellularly by the recombinant host, i.e. is not secreted by the cell, it may be recovered by standard procedures comprising cell disrupture by mechanical means, e.g. sonication or homogenization, or by enzymatic or chemical means to lowed by purification.

In order to be secreted, the DNA sequence encoding the polypeptide should be preceded by a sequence coding for a signal peptide, the presence of which ensures secretion of the polypeptide from the cells so that at least a significant proportion of the polypeptide expressed is secreted into the culture medium and recovered.

Another important aspect of the invention is a method of assaying for compounds which have the ability to inhibit the association of a sigma subunit to a *Mycobacterium tuberculosis* DNA polymerase, said method comprising the use of a recombinant SigB polypeptide or a nucleic acid molecule as defined above. Such a method will preferably comprise (i) contacting a compound to be tested for such inhibition ability with a r SigB polypeptide as described above and a *Mycobacterium tuberculosis* core RNA polymerase; and (ii) detecting whether the said polypeptide associates with the said core RNA polymerase to form RNA polymerase holoenzyme. The term "core RNA polymerase" is to be understood as an RNA polymerase which comprises at least the α, β, and β' subunits, but not the sigma subunit. The term "RNA polymerase holoenzyme" is to be understood as an RNA polymerase comprising at least the α, β, β' and sigma subunits. If desirable, the sigma subunit polypeptide can be labelled, for example with a suitable radioactive molecule, e.g. ³⁵S or ¹²⁵I.

Suitable methods for determining whether a sigma polypeptide has associated to core RNA polymerase are disclosed by Lesley et al. (Biochemistry 28, 7728-7734, 1989). Such a method may thus be based on the size difference between sigma polypeptide bound to core RNA polymerase, versus polypeptides not bound. This difference in size allows the two forms to be separated by chromatography, e.g. on a gel filtration column, such as a Waters Protein Pak^{®} 300SW sizing column. The two forms cluted from the column may be detected and quantified by known methods, such as scintillation counting or SDS-PAGE followed by immunoblotting.

According to another method also described by Lesley et al. *(supra),* RNA polymerase holoenzyme is detected by immunoprecipitation using an antibody binding to RNA polymerase holoenzyme. Core DNA polymerase from an organism such as *E*. *coli. M. tuberculosis* or *M. smegmatis* can be allowed to react with a radiolabelled SigA or SigB polypeptide. The reaction mix is treated with *Staphylococcus aureus* formalin-treated cell suspension, pretreated with an anti-RNA polymerase antibody. The cell suspension is washed to remove unbound proteins, resuspended in SDS-PAGE sample buffer and separated on SDS-PAGE. Bound SigB polypeptide are monitored by autoradiography followed by scintillation counting.

Another method of assaying for compounds which have the ability to inhibit sigma subunit-dependent transcription by a *Mycobacterium tuberculosis* RNA polymerase can comprise (i) contacting a compound to be tested for said inhibition ability with a polypeptide of the invention, a *Mycobacterium tuberculosis* core RNA polymerase, and a DNA having a coding sequence operably-linked to a promoter sequence capable of recognition by said core RNA polymerase when bound to said polypeptide, said contacting being carried out under conditions suitable for transcription of said coding sequence when *Mycobacterium tuberculosis* RNA polymerase is bound to said promoter; and (ii) detecting formation of mRNA corresponding to said coding sequence.

Such an assay is based on the fact that *E. coli* consensus promoter sequences are not transcribable by core RNA polymerase lacking the sigma subunit. However, addition of a sigma⁷⁰ protein will enable the complex to recognize specific promoters and initiate transcription. Screening of compounds which have the ability to inhibit sigma-dependent transcription can thus be performed, using DNA containing a suitable promoter as a template, by monitoring the formation of mRNA of specific length. Transcription can be monitored by measuring incorporation of ³H-UTP into TCA-precipitable counts (Ashok Kumar et al. (1994) J. Mol. Biol. 235, 405-413; Kajitani, M. and Ishihama, A. (1983) Nucleic Acids Res. 11, 671-686 and 3873-3888) and determining the length of the specific transcript. Compounds which are identified by such an assay can inhibit transcription by various mechanisms, such as (a) binding to a sigma protein and preventing its association with the core RNA polymerase; (b) binding to core RNA polymerase and sterically inhibiting the binding of a sigma protein; or (c) inhibiting intermediate steps involved in the initiation or elongation during transcription.

A further aspect of the invention is a method of determining the protein structure of a *Mycobacterium tuberculosis* RNA polymerase sigma subunit, characterised in that a SigB polypeptide is utilized in X-ray crystallography. The use of r SigB polypeptide in crystallisation will facilitate a rational design, based on X-ray crystallography, of therapeutic compounds inhibiting interaction of a sigma⁷⁰ protein with the core RNA polymerase, alternatively inhibiting the binding of a sigma70 protein, in association with a core RNA polymerase, to RNA during the course of gene transcription.

### EXAMPLES

### EXAMPLE 1: Identification of M. tuberculosis DNA sequences homologous to the sigma⁷⁰ gene

### 1.1. PCR amplification of putative sigma⁷⁰ homologues

The following PCR primers were designed, based on the conserved amino acid sequences of sigma⁴⁵ (a sigma⁷⁰ homologue) of *Bacillus subtilis* and sigma⁷⁰ of E. *coli* (Gitt, M.A. et al. (1985) J. Biol. Chem. 260, 7178-7185):

The alternative nucleotides indicated at certain positions indicate that the primers are regenerate primers suitable for amplification of the unidentified gene. tested for said inhibition ability with a polypeptide of the invention, a *Mycobacterium tuberculosis* core RNA polymerase, and a DNA having a coding sequence operably-linked to a promoter sequence capable of recognition by said core RNA polymerase when bound to said polypeptide, said contacting being carried out under conditions suitable for transcription of said coding sequence when *Mycobacterium tuberculosis* RNA polymerase is bound to said promoter; and (ii) detecting formation of mRNA corresponding to said coding sequence.

Such an assay is based on the fact that *E. coli* consensus promoter sequences are not transcribable by core RNA polymerase lacking the sigma subunit. However, addition of a sigma⁷⁰ protein will enable the complex to recognise specific promoters and initiate transcription. Screening of compounds which have the ability to inhibit sigma-dependent transcription can thus be performed, using DNA containing a suitable promoter as a template, by monitoring the formation of mRNA of specific lengths. Transcription can be monitored by measuring incorporation of ³H-UTP into TCA-precipitable counts (Ashok Kumar et al. (1994) J. Mol. Biol. 235, 405-413; Kajitani, M. and Ishihama, A. (1983) Nucleic Acids Res. 11, 671-686 and 3873-3888) and determining the length of the specific transcript. Compounds which are identified by such an assay can inhibit transcription by various mechanisms, such as (a) binding to a sigma protein and preventing its association with the core RNA polymerase; (b) binding to core RNA polymerase and sterically inhibiting the binding of a sigma protein; or (c) inhibiting intermediate steps involved in the initiation or elongation during transcription.

A further aspect of the invention is a method of determining the protein structure of a *Mycobacterium tuberculosis* RNA polymerase sigma subunit, characterised in that a SigA or SigB polypeptide is utilized in X-ray crystallography. The use of SigA or SigB polypeptide in crystallisation will facilitate a rational design, based on X-ray crystallography, of therapeutic compounds inhibiting interaction of a sigma⁷⁰ protein with the core RNA polymerase, alternatively inhibiting the binding of a sigma⁷⁰ protein, in association with a core RNA polymerase, to DNA during the course of gene transcription.

### EXAMPLES

### EXAMPLE 1: Identification of M. tuberculosis DNA sequences homologous to the sigma⁷⁰ gene

### 1.1. PCR amplification of putative sigma⁷⁰ homologues

The following PCR primers were designed, based on the conserved amino acid sequences of sigma⁴⁵ (a sigma⁷⁰ homologue) of *Bacillus subtilis* and sigma⁷⁰ of E. *coli* (Gitt, M.A. et al. (1985) J. Biol. Chem. 260, 7178-7185):

The alternative nucleotides indicated at certain positions indicate that the primers are degenerate primers suitable for amplification of the unidentified gene.

Chromosomal DNA from *M. tuberculosis* H37RV (ATCC 27294) was prepared following standard protocols. PCR amplification of a DNA fragment of approximately 500 bp was carried out using the following conditions:

| | | |
|---|---|---|
| Annealing: | +55°C | 1 min |
| Denaturation: | +93°C | 1 min |
| Extension: | +73°C | 2 min |

### 1.2. Southern hybridisation of M. tuberculosis DNA

Chromosomal DNA from *M. tuberculosis* H37RV (ATCC 27294), *M. tuberculosis* H37RA and *Mycobacterium smegmatis* was prepared following standard protocols and restricted with the restriction enzyme *Sal*I. The DNA fragments were resolved on a 1% agarose gel by electrophoresis and transferred onto nylon membranes which were subjected to "Southern blotting" analysis following standard procedures. To detect homologous fragments, the membranes were probed with a radioactively labelled -500 bp DNA fragment, generated by PCR as described above.

Analysis of the Southern hybridisation experiment revealed the presence of at least three hybridising fragments of approximately 4.2, 2.2 and 0.9 kb, respectively, in the *Sal*I-digested DNA of both of the *M. tuberculosis* strains. In *M. smegmatis,* two hybridising fragments of 4.2 and 2.2 kb, respectively, were detected. It could be concluded that there were multiple DNA fragments with homology to the known sigma⁷⁰ genes.

Similar Southern hybridisation experiments, performed with four different clinical isolates of *M. tuberculosis,* revealed identical patterns, indicating the presence of similar genes also in other virulent isolates of *M. tuberculosis.*

### EXAMPLE 2: Cloning of putative sigma⁷⁰ homologues

### 2.1. Cloning of M. tuberculosis sigA

A lambda gt11 library (obtained from WHO) of the chromosomal DNA of *M. tuberculosis* Erdman strain was screened, using the 500 bp PCR probe as described above, following standard procedures. One lambda gt11 phage with a 4.7 kb *Eco*RI insert was identified and confirmed to hybridise with the PCR probe. Restriction analysis of this 4.7 kb insert revealed it to have an internal 2.2 kb *Sal*I fragment which hybridised with the PCR probe.

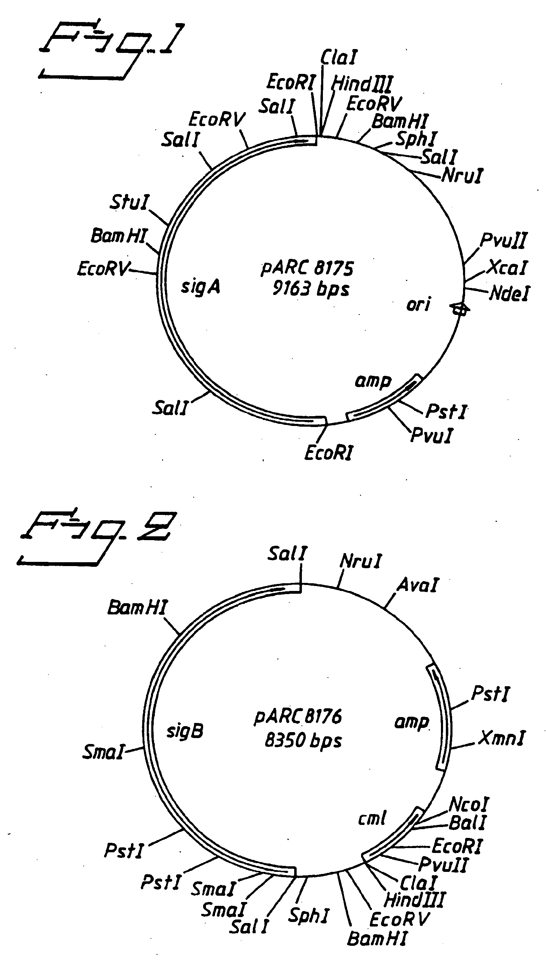

The 4.7 kb fragment was excised from the lambda gt 11 DNA by *Eco*RI restriction, and subcloned into the cloning vector pBR322, to obtain the recombinant plasmid pARC 8175 (Fig. 1) (NCIMB 40738).

The putative sigma⁷⁰ homologue on the 2.2 kb *Sal*I fragment was designated *M. tuberculosis sigA.* The coding sequence of the *sigA* gene was found to have an internal *Sal*I site, which could explain the hybridisation of the 0.9 kb fragment in the Southern experiments.

### 2.2. Cloning of M. tuberculosis sigB

*M. tuberculosis* H37Rv DNA was restricted with *Sal*I and the DNA fragments were resolved by preparative agarose gel electrophoresis. The agarose gel piece corresponding to the 4.0 to 5.0 kb size region was cut out, and the DNA from this gel piece was extracted following standard protocols. This DNA was ligated to the cloning vector pBR329 at its *Sal*I site, and the ligated DNA was transformed into *E. coli* DH5α to obtain a sub-library. Transformants of this sub-library were identified by colony blotting, using the PCR-derived 500 bp probe, following standard protocols. Individual transformant colonies were analyzed for their plasmid profile. One of the recombinant plasmids retaining the expected plasmid size, was analyzed in detail by restriction mapping and was found to harbour the expected 4.2 kb *Sal*I DNA fragment. This plasmid with the *sigB* gene on the 4.2 kb insert was designated pARC 8176 (Fig. 2) (NCIMB 40739).

### EXAMPLE 3: Nucleotide sequence of M. tuberculosis sigA and sigB genes

### 3.1. Nucleotide sequence of sigA

The *Eco*RV *-* EcoRI DNA fragment expected to encompass the entire *sigA* gene was subcloned into appropriate M13 vectors and both strands of the gene sequenced by the dideoxy method. The sequence obtained is shown as SEQ ID NO: 1 in the Sequence Listing. An open reading frame (ORF) of 1580 nucleotides (positions 70 to 1650 in SEQ ID NO: 1) coding for a protein of 526 amino acids was predicted from the DNA sequence. The N-terminal amino acid has been assigned tentatively based on the first GTG (initiation codon) of the ORF.

The derived amino add sequence of the gene product SigA (SEQ ID NO: 2) showed 60% identity with the *E. coli* sigma⁷⁰ and 70% identity with the HrdB sequence of *Streptomyces coelicolor.* The overall anatomy of the SigA sequence is compatible with that seen among sigma⁷⁰ proteins of various organisms. This anatomy comprises a highly conserved C-terminal half, while the N-terminal half generally shows lesser homology The two regions are linked by a stretch of amino acids which varies in length and is found to be generally unique for the protein. The SigA sequence has a similar structures where the unconserved central stretch correspond to amino acids 270 to 306 in SEQ ID NO: 2.

The N-terminal half has limited homology to *E. coli* sigma⁷⁰, but shows resemblance to that of the sigma⁷⁰ homologue HrdB of *S. coelicolor.* The highly conserved motifs of regions 3.1, 3.2, 4.1 and 4.2 of *S. coelicolor* which were proposed to be involved in DNA binding (Lonetto, M. et al. (1992) J. Bacteriol. 174, 3843-3849) are found to be nearly identical also in the *M. tuberculosis* SigA sequence. The N-terminal start of the protein has been tentatively assigned, based on homologous motifs of the *S. coelicolor* HrdB sequence.

The overall sequence similarity of the SigA and SigB amino acid sequences to known sigma⁷⁰ sequences suggests assignment of the *M. tuberculosis* SigA to the Group I sigma⁷⁰ proteins. However, SigA also shows distinct differences with known sigma⁷⁰ proteins, in particular a unique and lengthy N-terminal stretch of amino acids (positions 24 to 263 in SEQ ID NO: 2), which may be essential for the recognition and initiation of transcription from promoter sequences of *M. tuberculosis.*

### 3.2. Nucleotide sequence of sigB

The nucleotide sequence of the *sigB* gene (SEQ ID NO: 3) encodes a protein of 323 amino adds (SEQ ID NO: 4). The N-terminal start of the protein has been tentatively identified based on the presence of the first methionine of the ORF. The ORF is thus estimated to start at position 325 and to end at 1293 in SEQ ID NO: 3. Alignment of the amino add sequence of the *sigB* gene with other sigma⁷⁰ proteins places the *sigB* gene into the Group I family of sigma⁷⁰ proteins. The overall structure of the gene product SigB follows the same pattern as described for SigA. However, the SigB sequence has only 60% homology with the SigA sequence, as there are considerable differences not only within the unconserved regions of the protein, but also within the putative DNA binding regions of the sigB protein. These characteristics suggest that the SigB protein may play a distinct function in the physiology of the organism.

### EXAMPLE 4: Expression of sigA and sigB

### 4.1. Expression of M. tuberculosis sigA gene in E. coli

The N-terminal portion of the *sigA* gene was amplified by PCR using the following primers: Reverse primer (SEQ ID NO: 8):
5'-GTA CAG GCC AGC CTC GAT CCG CTT GGC-3'
(a) A fragment of approximately 750 bp was amplified from the *sigA* gene construct pARC 8175. The amplified product was restricted with *Nco*I and *Bam*HI to obtain a 163 bp fragment.
(b) A 1400 bp DNA fragment was obtained by digestion of pARC 8175 with *Bam*HI and *Eco*RV.
(c) The expression plasmid pET 8ck, which is a derivative of pET 8c (Studier, F.W. et al. (1990) Methods Enzymol. 185, 61-89) in which the β-lactamase gene has been replaced by the gene conferring kanamycin resistance, was digested with *Nco*I and *Eco*RV and a fragment of approximately 4.2 kb was purified.

These three fragments (a), (b) and (c) were ligated by standard methods and the product was transformed into *E. coli* DH5α. Individual transformants were screened for the plasmid profile following standard protocols. The transformant was identified based on the expected plasmid size (approximately 6.35 kb) and restriction mapping of the plasmid. The recombinant plasmid harbouring the coding fragment of *sigA* was designated pARC 8171.

The plasmid pARC 8171 was transformed into the T₇ expression host *E. coli* BL21(DE3). Individual transformants were screened for the presence of the 6.35 kb plasmid and confirmed by restriction analysis. One of the transformants was grown at 37°C and induced with 1 mM isopropyl-β-D-thiogalactopyranoside (IPTG) using standard protocols. A specific 90 kDa protein was induced on expression. Cells were harvested by low speed centrifugation and lysed by sonication in phosphate buffered saline, pH 7.4. The lysate was centrifugated at 100,000 x g to fractionate into supernatant and pellet. The majority of the 70 kDa product obtained after induction with IPTG was present in the pellet fraction, indicating that the protein formed inclusion bodies.

For purifying the induced *sigA* gene product, the cell lysate as obtained above was clarified by centrifugation at 1000 rpm in Beckman JA 21 rotor for 15 min. The clarified supernatant was layered on a 15-60% sucrose gradient and centrifugated at 100,000 x g for 60 min. The inclusion bodies sedimented as a pellet through the 60% sucrose cushion. This pellet was solubilised in 6 M guanidine hydrochloride which was removed by sequential dialysis against buffer containing decreasing concentration of guanidine hydrochloride. The dialysate was 75% enriched for the SigA protein which was purified essentially following the protocol for purification *E. coli* sigma⁷⁰ as described by Brokhov, S. and Goldfarb, A. (1993) Protein expression and purification, vol. 4, 503-511.

### 4.2. Expression of M. tuberculosis sigB gene in E. coli

The *sigB* gene product was expressed and purified from inclusion bodies. The coding sequence of the *sigB* gene was amplified by PCR using the following primers: Reverse primer (SEQ ID NO: 10), comprising an EcoRI restriction site:
5'- CTT GAA TTC AGC TGG CGT ACG ACC GCA-3'

The amplified 920 bp fragment was digested with EcoRI and *Nco*I and ligated to the *Eco*RI- and *Nco*I-digested pRSET B (Kroll et al. (1993) DNA and Cell Biology 12, 441). The ligation mix was transformed into *E. coli* DH5α. Individual transformants were screened for plasmid profile and restriction analysis. The recombinant plasmid having the expected plasmid profile was designated pARC 8193.

*E. coli* DH5α harbouring pARC 8193 was cultured in LB containing in 50 µg/ml ampicillin till an OD of 0.5, and induced with 1 mM IPTG at 37°C, following standard protocols. The induced SigB protein was obtained as inclusion bodies which were denatured and renatured following the same protocol as described for the SigA protein. The purified SigB protein was >90% homogenous and suitable for transcription assays.

### DEPOSIT OF MICROORGANISMS

The following plasmids have been deposited under the Budapest Treaty at the National Collections of Industrial and Marine Bacteria (NCIMB), Aberdeen, Scotland, UK.

| Plasmid | Accession No. | Date of deposit |
|---|---|---|
| pARC 8175 | NCIMB 40738 | 15 June 1995 |
| pARC 8176 | NCIMB 40739 | 15 June 1995 |

## Claims

1. An isolated polypeptide which is a Group I sigma subunit of *Mycobacterium tuberculosis* RNA polymerase, whose amino acid sequence is identical to, or at least 95% homologous with SEQ ID NO: 4 in the Sequence Listing.

2. An isolated nucleic acid molecule which has a nucleotide sequence coding for a polypeptide as claimed in claim 1 and which molecule is selected from:
(a) DNA molecules comprising a nucleotide sequence as shown in SEQ ID NO: 3 encoding a Group I sigma subunit of *Mycobacterium tuberculosis* RNA polymerase;
and
(b) nucleic acid molecules comprising a nucleic acid sequence which is degenerate, as a result of the genetic code, to a nucleotide sequence as defined in (a) and which codes for a polypeptide which is a Group I sigma subunit of *Mycobacterium tuberculosis* RNA polymerase.

3. A vector which comprises a nucleic acid molecule according to claim 2.

4. A vector according to claim 3 which is the plasmid vector pARC 8176 (NCIMB 40739).

5. A vector according to claim 3 which is an expression vector capable of mediating the expression of a polypeptide according to claim 1.

6. A host cell harbouring a vector according to any one of claims 3 to 5.

7. A process for production of a polypeptide according to claim 1 which comprises culturing a host cell according to claim 6 transformed with an expression vector according to claim 5 under conditions whereby said polypeptide is produced and recovering said polypeptide.

8. A method of assaying for compounds which have the ability to inhibit the association of a sigma subunit with a *Mycobacterium tuberculosis* core RNA polymerase, said method comprising (i) contacting a compound to be tested for said inhibition ability with a polypeptide according to claim land a *Mycobacterium tuberculosis* core RNA polymerase; and (ii) detecting whether the said polypeptide associates with the said core RNA polymerase to form RNA polymerase holoenzyme.

9. A method according to claim 8 wherein polypeptides which are associated to core RNA polymerase and / or polypeptides which are not associated to core RNA polymerase are detected by chromatography such as gel filtration.

10. A method according to claim 8 wherein RNA polymerase holoenzyme is detected by immunoprecipitation, using an antibody binding to RNA polymerase holoenzyme.

11. A method of assaying for compounds which have the ability to inhibit sigma subunit-dependent transcription by a *Mycobacterium tuberculosis* RNA polymerase, said method comprising (i) contacting a compound to be tested for said inhibition ability with a polypeptide according to claim 1, a *Mycobacterium tuberculosis* core RNA polymerase, and a DNA having a coding sequence operably-linked to a promoter sequence capable of recognition by said core RNA polymerase when bound to said polypeptide, said contacting being carried out under conditions suitable for transcription of said coding sequence when *Mycobacterium tuberculosis* RNA polymerase is bound to said promoter; and (ii) detecting formation of mRNA corresponding to said coding sequence.

12. A method of determining the protein structure of a *Mycobacterium tuberculosis* RNA polymerase sigma subunit, **characterised in that** a polypeptide according to claim 1 is utilized in X-ray crystallography.

## Patentansprüche

1. Isoliertes Polypeptid, bei dem es sich um eine Gruppe-I-sigma-Untereinheit der RNA-Polymerase aus *Mycobacterium tuberculosis* handelt, deren Aminosäuresequenz zu der SEQ ID NO: 4 im Sequenzprotokoll identisch oder wenigstens zu 95% homolog ist.

2. Isoliertes Nukleinsäuremolekül, das eine für ein Polypeptid nach Anspruch 1 codierende Nukleotidsequenz aufweist und das ausgewählt ist aus:
(a) DNA-Molekülen, die eine wie in der SEQ ID NO: 3 dargestellte und für eine Gruppe-I-sigma-Untereinheit der RNA-Polymerase aus *Mycobacterium tuberculosis* codierende Nukleotidsequenz umfassen, und
(b) Nukleinsäuremolekülen, die eine Nukleinsäuresequenz umfassen, welche als Ergebnis des genetischen Codes zu einer Nukleotidsequenz mit der unter (a) angegebenen Bedeutung degeneriert ist und für ein Polypeptid codiert, bei dem es sich um eine Gruppe-I-sigma-Untereinheit aus *Mycobacterium tuberculosis* handelt.

3. Vektor, der ein Nukleinsäuremolekül gemäß Anspruch 2 umfaßt.

4. Vektor gemäß Anspruch 3, bei dem es sich um den Plasmidvektor pARC 8176 (NCIMB 40739) handelt.

5. Vektor gemäß Anspruch 3, bei dem es sich um einen zur Vermittlung der Expression eines Polypeptids gemäß Anspruch 1 fähigen Expressionsvektor handelt.

6. Wirtszelle, enthaltend einen Vektor gemäß einem der Ansprüche 3 bis 5.

7. Verfahren zur Herstellung eines Polypeptids gemäß Anspruch 1, bei dem man eine mit einem Expressionsvektor gemäß Anspruch 5 transformierte Wirtszelle unter Bedingungen, bei denen das Polypeptid produziert wird, kultiviert und das Polypeptid gewinnt.

8. Verfahren zum Testen auf Verbindungen, die die Fähigkeit zur Hemmung der Assoziation einer sigma-Untereinheit mit einer Kern-RNA-Polymerase aus *Mycobacterium tuberculosis* aufweisen, wobei man in dem Verfahren (i) eine auf die Hemmfähigkeit zu testende Verbindung mit einem Polypeptid gemäß Anspruch 1 sowie einer Kern-RNA-Polymerase aus *Mycobacterium tuberculosis* in Kontakt bringt und (ii) nachweist, ob das Polypeptid mit der Kern-RNA-Polymerase unter Bildung des RNA-Polymerase-Holoenzyms assoziiert.

9. Verfahren gemäß Anspruch 8, wobei Polypeptide, die an Kern-RNA-Polymerase angelagert sind, und/oder Polypeptide, die nicht an Kern-RNA-Polymerase angelagert sind, mittels Chromatographie, wie etwa Gelfiltration, nachgewiesen werden.

10. Verfahren gemäß Anspruch 8, wobei RNA-Polymerase-Holoenzym mittels Immunpräzipitation unter Verwendung eines an RNA-Polymerase-Holoenzym bindenden Antikörpers nachgewiesen wird.

11. Verfahren zum Testen auf Verbindungen, die die Fähigkeit zur Hemmung der sigma-Untereinheitabhängigen Transkription durch eine Kern-RNA-Polymerase aus *Mycobacterium tuberculosis* aufweisen, wobei man in dem Verfahren (i) eine auf die Hemmfähigkeit zu testende Verbindung mit einem Polypeptid gemäß Anspruch 1, einer Kern-RNA-Polymerase aus *Mycobacterium tuberculosis* sowie einer DNA mit einer in operativer Verknüpfung mit einer Promotorsequenz, die von der Kern-RNA-Polymerase bei Bindung an das Polypeptid erkannt werden kann, stehenden codierenden Sequenz in Kontakt bringt, wobei das Inkontaktbringen unter für die Transkription der codierenden Sequenz geeigneten Bedingungen bei Bindung von RNA-Polymerase aus *Mycobacterium tuberculosis* an den Promotor erfolgt und (ii) die Bildung von der codierenden Sequenz entsprechender mRNA nachweist.

12. Verfahren zur Bestimmung der Proteinstruktur einer sigma-Untereinheit der RNA-Polymerase aus Myco*bacterium tuberculosis,* **dadurch gekennzeichnet, daß** bei der Röntgenkristallographie ein Polypeptid gemäß Anspruch 1 eingesetzt wird.

## Revendications

1. Polypeptide isolé qui est une sous-unité sigma de groupe I d'ARN polymérase de *Mycobacterium tuberculosis,* dont la séquence d'acides aminés est identique, ou homologue à au moins 95 % de SEQ ID NO : 4 dans le listage de séquences.

2. Molécule d'acide nucléique isolée qui a une séquence de nucléotides codant pour un polypeptide tel que revendiqué dans la revendication 1 et dont la molécule est sélectionnée à partir :
(a) de molécules d'ADN comprenant une séquence de nucléotides telle que montrée dans SEQ ID NO : 3 codant pour une sous-unité sigma de groupe I d'ARN polymérase de *Mycobacterium* tuberculosis ;
et
(b) de molécules d'acide nucléique comprenant une séquence d'acides nucléiques qui est dégénérée, du fait du code génétique, en une séquence de nucléotides telle que définie dans (a) et qui code pour un polypeptide qui est une sous-unité sigma de groupe I de *Mycobacterium tuberculosis.*

3. Vecteur qui comprend une molécule d'acide nucléique selon la revendication 2.

4. Vecteur selon la revendication 3 qui est le vecteur plasmidique pARC 8176 (NCIMB 40739).

5. Vecteur selon la revendication 3 qui est un vecteur d'expression capable d'engendrer l'expression d'un polypeptide selon la revendication 1.

6. Cellule hôte portant un vecteur selon l'une quelconque des revendications 3 à 5.

7. Procédé de production d'un polypeptide selon la revendication 1 qui comprend la mise en culture d'une cellule hôte selon la revendication 6 transformée avec un vecteur d'expression selon la revendication 5 dans des conditions dans lesquelles ledit polypeptide est produit et la récupération dudit polypeptide.

8. Procédé d'essai de composés qui ont la capacité d'inhiber l'association d'une sous-unité sigma avec une ARN polymérase coeur de *Mycobacterium tuberculosis,* ledit procédé comprenant (i) la mise en contact d'un composé dont on doit tester ladite capacité d'inhibition avec un polypeptide selon la revendication 1 et une ARN polymérase coeur de *Mycobacterium tuberculosis,* et (ii) le fait de détecter si ledit polypeptide s'associe avec ladite ARN polymérase coeur pour former une holoenzyme ARN polymérase.

9. Procédé selon la revendication 8 dans lequel des polypeptides qui sont associés à une ARN polymérase coeur et/ou des polypeptides qui ne sont pas associés à une ARN polymérase coeur sont détectés par chromatographie telle que par filtration sur gel.

10. Procédé selon la revendication 8 dans lequel une holoenzyme ARN polymérase est détectée par immuno-précipitation, en utilisant un anticorps se liant à une holoenzyme ARN polymérase.

11. Procédé d'essai de composés qui ont la capacité d'inhiber la transcription dépendant de la sous-unité sigma par une ARN polymérase de *Mycobacterium tuberculosis,* ledit procédé comprenant (i) la mise en contact d'un composé dont on doit tester ladite capacité d'inhibition avec un polypeptide selon la revendication 1, une ARN polymérase coeur de *Mycobacterium tuberculosis,* et un ADN ayant une séquence codante liée de manière fonctionnelle à une séquence promotrice capable d'une reconnaissance par ladite ARN polymérase coeur lorsque liée audit polypeptide, ladite mise en contact étant réalisée dans des conditions adaptées pour une transcription de ladite séquence codante lorsqu'une ARN polymérase de *Mycobacterium tuberculosis* est liée audit promoteur ; et (ii) le fait de détecter une formation d'ARNm correspondant à ladite séquence codante.

12. Procédé de détermination de la structure protéique d'une sous-unité sigma d'ARN polymérase de *Mycobacterium tuberculosis,* **caractérisé en ce qu'**un polypeptide selon la revendication 1 est utilisé dans une cristallographie par rayons X.
